# EUROPEAN PATENT APPLICATION

(11) **EP 3 763 746 A1**
(43) Date of publication of application: **13.01.2021**
(21) Application number: 19185375.3
(22) Date of filing: 10.07.2019
(51) Int. Cl.: C08F 2/22, A61K 8/06, C08F 2/24, C08F 2/30, C08F 220/18

(54) **METHOD FOR PREPARING MINIEMULSIONS COMPRISING SUPERHYDROPHOBIC MONOMERS**

(71) Applicant: Clariant International Ltd, 4132 Muttenz (CH)
(72) Inventor: PITARCH LOPEZ, Jesus, 60431 Frankfurt am Main (DE); KLEINBLOTEKAMP, Jochen, 63303 Dreieich (DE); SAHL, Mike, 65520 Bad Camberg (DE); STOPPEL, Yasemin, 65929 Frankfurt am Main (DE)
(74) Representative: Paczkowski, Marcus

(57) **Abstract**

The invention relates to a method for preparing oil-in-water miniemulsions comprising monomer droplets with a mean diameter in the range of from 50 to 1000 nm, wherein the monomer droplets comprising superhydrophobic or hydrophobic monomers. The method comprises the addition of adding an oil phase to an emulsifier system, wherein the oil phase comprises at least 5 wt.-% to 100 wt.-% of one or more superhydrophobic monomers or hydrophobic monomers and 0 wt.-% to 95 wt.-% of non-superhydrophobic monomers and the emulsifier system comprises at least one non-ionic surfactant selected from the group of saturated branched alcohols having 7 to 30 carbon atoms which are alkoxylated with 4 to 20 alkyloxy groups, or a mixture of surfactants consisting of at least 20 wt.-% and less than 100 wt.-% of the at least one non-ionic surfactant and more than 0 wt.-% to 80 wt.-% of at least one anionic surfactant, and water, until gel formation is observed, and diluting the obtained gel with an aqueous phase.

Further embodiments are miniemulsions prepared by the described method and their use for the preparation of polymer dispersions.

## Description

### Field of the invention:

The invention relates to a method for preparing oil-in-water miniemulsions comprising monomer droplets with a mean diameter in the range of from 50 to 1000 nm, wherein the monomer composition forming the droplets comprises superhydrophobic monomers. Further embodiments of the invention are miniemulsions prepared by the described method and their use for the preparation of polymer dispersions.

### Background of the invention:

Emulsion polymerization is a widely used process for the production of aqueous polymer dispersions. An emulsion polymerisation is a radical polymerization carried out in aqueous monomer dispersions. The most common type of emulsion polymerization is the polymerisation of oil-in-water emulsions, in which droplets of monomer (oil phase) are emulsified with surfactants in a continuous phase of water. The monomer dispersion comprises relatively large droplets of monomer and relatively small micelles of excess surfactant. Small amounts of monomer diffuse through the water to the small micelles where the monomers are polymerised by a water-soluble initiator. The final product is a dispersion of polymer particles in water. It can also be known as a polymer, latex, or polymer emulsion.

A crucial requirement for emulsion polymerisations is the monomer transport through the aqueous phase. A certain water-solubility of the monomers is required to allow diffusion from large monomer droplets to the small micelles, wherein polymerisation takes place. Vice versa large monomer droplets are not detrimental for the preparation of a dispersion of polymer nanoparticles or a latex dispersion. Monomer compositions for the preparation of latex dispersions comprising large droplets of monomers with sufficient water-solubility can be easily obtained by stirring the monomers in the presence of surfactants or protective colloids.

However, common emulsion polymerisation methods are not suitable for the polymerisation of superhydrophobic monomers having very low water-solubility. For the preparation of dispersions comprising polymer nanoparticles derived from superhydrophobic monomers miniemulsion polymerisation methods are developed wherein the polymerisation takes place directly in the monomer droplets. Therefore monomer emulsions are required comprising submicron monomer droplets.

A common technique of preparing miniemulsions is to break up the droplets of a coarse emulsion produced by conventional agitation to submicron size droplets using high energy devices, such as sonicators, high pressure homogenizers or high shear mixers. An implementation of such methods at an industrial scale is difficult because special equipment is required for miniemulsification and energy consumption is high (Asua, Jose M., Challenges for industrialization of miniemulsion polymerization, Progress in Polymer Science 39 (2014), 1797-1826). Moreover, the time to achieve the required small monomer droplet size at large scale is typically long, which impacts negatively the economically viability of the overall manufacture process of the polymer dispersion.

Further methods have been reported to achieve emulsion polymerization of superhydrophobic monomers, comprising the use of either toxic solvents, phase transfer agents, such as cyclodextrines, or polymeric seeds.

A further method for the preparation of miniemulsions is described by Bascán, F. et al. (From miniemulsion to nanoemulsion polymerization of superhydrophobic monomers through low energy phase inversion temperature, Journal of Industrial and Engineering Chemistry 58 (2018) 1-8). According to Bascán, F. et al. monomer miniemulsions are obtainable by heating a coarse oil-in-water emulsion comprising a superhydrophobic monomer until phase inversion to a water-in-oil emulsion is achieved. Phase inversion is detected by a sharp decrease of the conductivity of the emulsion. After phase inversion the emulsion was cooled, returning to an oil-in-water miniemulsion. Typical phase inversion temperatures for appropriate non-ionic emulsifiers are in the range of 60 °C to 90 °C. The upper limit is established by the boiling point of the continuous phase, the lower limit is set by the reaction temperature, which must be lower than the phase inversion temperature (PIT).

A further phase inversion method for the formation of miniemulsions is described by Elgammal M., Preparation of latex nanoparticles using nanoemulsion obtained by the phase inversion composition (PIC) method and their application in textile printing, Colloids and Surfaces A: Physicochem. Eng. Aspects 470, (2015), 70-79.

The described methods are particularly suitable for the preparation of miniemulsions in laboratory scale. Therefore, it is an object of the present invention to provide an improved method for the preparation of miniemulsions suitable for upscaling for industrial applications.

### Description of the invention:

The problem is solved by a method for preparing miniemulsions of superhydrophobic monomers and/or hydrophobic monomers, wherein a monomer composition comprising the monomers is added to an aqueous emulsifier system comprising at least one non-ionic surfactant selected from the group of saturated branched alcohols having 7 to 25 carbon atoms which are alkoxylated with 4 to 20 alkyloxy groups selected from the group consisting of ethylene oxide, propylene oxide and butylene oxide or mixtures thereof, or a mixture of surfactants consisting of at least 20 wt.-% and less than 100 wt.-% of the at least one non-ionic surfactant and more than 0 wt.-% to 80 wt.-% of at least one anionic surfactant at temperatures between 0° and 50°C, until gel formation is observed. Then, the obtained gel is diluted with an aqueous phase to obtain the desired miniemulsion, which comprises monomer droplets with the desired mean diameter in the range of from 50 nm to 800 nm. The obtained miniemulsions can be stored or directly used for the preparation of corresponding polymer dispersions.

Therefore a first embodiment of the invention is a method for preparing an oil-in-water miniemulsion comprising monomer droplets with a mean diameter in the range of from 50 to 1000 nm, preferably from 50 nm to 800 nm, more preferably from 50 to 500 nm and particularly preferred from 100 to 300 nm wherein the method comprises the steps of
(a) stirring an emulsifier system comprising
   (i) at least one non-ionic surfactant selected from the group of saturated branched alcohols having 7 to 30 carbon atoms which are alkoxylated with 4 to 20 alkyloxy groups selected from the group consisting of ethylene oxide, propylene oxide and butylene oxide or mixtures thereof, or a mixture of surfactants consisting of at least 20 wt.-% and less than 100 wt.-% of the at least one non-ionic surfactant and more than 0 wt.-% to 80 wt.-% of at least one anionic surfactant, and
   (ii) water,
   and adjusting the temperature of the emulsifier system to a range between 0°C and 50 °C,
(b) adding an oil phase to the emulsifier system (a), wherein the oil phase comprises at least 5 wt.-% to 100 wt.-% of one or more superhydrophobic monomers and/or hydrophobic monomers and 0 wt.-% to 95 wt.-% of non-superhydrophobic monomers, until gel formation occurs, and
(c) diluting the gel obtained in step (b) with an aqueous phase under stirring at a temperature between 0 °C and 50 °C, wherein a miniemulsion comprising the superhydrophobic monomers is obtained.

Preferred non-ionic surfactants are selected from the group consisting of branched (C₈-C₂₆)-alkanols having a terminal hydroxyl group which is alkoxylated with 4 to 20 alkoxy groups selected from ethylene oxide, propylene oxide and butylene oxide. The branched alkanoyl residue comprises preferably one or more side groups, preferably up to five alkyl side groups, with up to 4 carbon atoms. Preferred alkyl side groups are selected from the group consisting of methyl, ethyl, n-propyl, i-propyl, n-butyl, sec.-butyl and tert.-butyl. Preferably the branched alkanoyl group has 9 to 21 carbon atoms with one, two or three side groups selected from the group consisting of methyl and ethyl.

Particularly preferred are non-ionic surfactants of formula (I)

H-(OA)ₘ-O-(CR'₂)ₙ-CR₃ (I)

wherein
one or two of the residues R' and/or at least two of the residues R are independently methyl, ethyl, propyl or butyl and the other residues R and R' are hydrogen, and wherein the number of carbon atoms of the -(CR'₂)ₙ-CR₃ is from 7 to 30, and wherein the groups (OA) are selected from the group consisting of ethylene oxide, propylene oxide, butylene oxide and mixtures thereof, and wherein
- m: is an integer from 4 to 20, preferably from 4 to 12, more preferred from 6 to 10 and most preferred from 6 to 8, and
- n: is an integer from 7 to 16, preferably from 9 to 13.

Particularly preferred are non-ionic surfactant is selected from a compound of formula (II)

H-(O-C₂H₄)ₘ-O-(CH₂)ₙ-CHR₂ (II)

wherein
both residues R are independently methyl or ethyl,
- m: is an integer from 4 to 12, preferably from 6 to 12, more preferably from 6 to 10 and most preferred from 6 to 8, and
- n: is an integer from 7 to 16, preferably from 9 to 13.

In one embodiment of in the inventive method the emulsifier system is free of anionic and/or cationic surfactants. But in a preferred embodiment of the inventive method the emulsifier system comprises additionally anionic surfactants. The anionic surfactants are preferably selected from the group of phosphate esters of alkoxylated aliphatic or aromatic alcohols. Particularly anionic surfactants selected from the group of phosphate esters of compounds of formula (III)

H-(OA)ₘ-O-(CR₂)ₙ-CR₃ (III)

wherein
one or two of the residues R are a methyl or ethyl group and the other residues R are hydrogen or all of the residues R are hydrogen, the groups (OA) are selected from the group of ethylene oxide, propylene oxide, butylene oxide and mixtures thereof, and
- m: is an integer from 2 to 20, and
- n: is an integer from 5 to 21.

If the emulsifier system comprises a mixture of non-ionic surfactant and anionic surfactant, the amount of anionic surfactant, based on the total amount of surfactant in the emulsifier system, is preferably from 10 wt.-% to 80 wt.-%, particularly from 15 wt.-% to 35 wt.-%, and the amount of non-ionic surfactant, based on the total amount of surfactant in the emulsifier system, is preferably from 20 wt.-% to 90 wt.-%, particularly from 65 wt.-% to 85 wt.-%.

In an alternative embodiment the amount of anionic surfactant, based on the total amount of surfactant in the emulsifier system, is from 65 wt.-% to 80 wt.-%, and the amount of non-ionic surfactant, based on the total amount of surfactant in the emulsifier system, is from 20 wt.-% to 35 wt.-%.

The emulsifier system described herein is particularly useful for providing a miniemulsion of superhydrophobic and hydrophobic monomers. The emulsifier system surprisingly allows the preparation of oil-in-water miniemulsions of superhydrophobic monomers without using high shear stirring, high pressure homogenization and without heating to phase inversion (PIT). Moreover, the emulsifier system is incorporated in the aqueous phase and not in the monomer phase like in the PIC process. In addition the emulsification can be carried out in aqueous phase in the absence of toxic solvents and in the absence of phase transfer agents.

The emulsifier system optionally comprises with water miscible co-solvents. Suitable co-solvents are for example methanol, ethanol, propanol, butanol, benzyl alcohol, ethylene glycol, di-ethylene glycol, propylene glycol, di-propylene glycol, glycerol, polyethylene glycol, polypropylene glycol, and mono-methyl ether or di-methyl ether of ethylene glycol or propylene glycol.

The amount of water in the emulsifier system is at least 10 wt.-% and up to 90 wt.-%, preferably from 20 wt.-% to 60 wt.-%. If a water miscible co-solvent is added to the emulsifier system, the amount of the co-solvent is usually in the range of from 10 wt.-% to 50 wt.-%, preferably of from 15 wt.-% to 35 wt.-% and the amount of water is preferably from 10 wt.-% to 60 wt.-% and more preferably from 20 wt.-% to 40 wt.-% based on the total amount of the emulsifier system. The water-miscible co-solvent is typically present in an amount of less than 50 wt.-%, preferably less than 35 wt.-% based on the total amount of the emulsifier system.

The total amount of non-ionic and anionic surfactant in the emulsifier system is usually in the range of from 10 wt.-% to 90 wt.-%, particularly in the range of from 20 wt.-% to 60 wt.-%.

In a preferred embodiment the emulsifier system consists of the non-ionic surfactant selected from the group of saturated branched alcohols having 7 to 30 carbon atoms which are alkoxylated with 4 to 20 alkyloxy groups selected from the group consisting of ethylene oxide, propylene oxide and butylene oxide or mixtures thereof, optionally an anionic surfactant,
optionally a water-miscible co-solvent, and
water.

In another embodiment of the described method further surfactants which are different from the non-ionic-surfactant described above can be added to the emulsifier system in an amount of less than 20 wt.-%, preferably less than 10 wt.-% or less than 5 wt.-%, based on the total amount of the emulsifier system.

These additional surfactants may be nonionic, anionic, cationic or amphoteric surfactants. Examples of suitable nonionic surfactants are polyoxyethylene sorbitan esters, polyoxyethylene sorbitol esters, polyoxyalkylene fatty alcohol ethers, polyoxyalkylene fatty acid esters, alkoxylated glycerides, polyoxyethylene methyl glucoside ester, alkyl polyglucosides, EO-PO blockpolymers or combinations thereof.

Examples of anionic surfactants are sulfonates of alkylbenzene-sulfonates, alkanesulfonates, olefinsulfonates, alkyl ether sulfate, alkyl sulfate, sulfo-succinates, alkyl phosphates, alkyl ether phosphates, protein fatty acid condensates, preferably collagen hydrolysates modified with fatty acid, amino acid-based surfactants, isethionates, taurides, acyl lactylates, neutralized fatty acids or combinations thereof.

Examples of cationic surfactants are esterquats, ditallow dimethyl ammonium chloride, C₁₂/₁₄ alkyl dimethyl benzyl ammonium chloride, alkyl dimethyl benzyl ammonium chloride, cetyl trimethyl ammonium chloride, stearyl trimethyl ammonium chloride, behenyl trimethyl ammonium chloride alkyl hydroxyethyl dimethyl ammonium chloride, distearyl dimethyl ammonium chloride, dihydrogenated tallow fatty alkyl dimethyl ammonium chloride or combinations thereof.

Examples of amphoteric surfactants are alkyl amphoacetate, alkyl amidopropyl betaine, alkyl amidopropyl dimethylamine betaine, undecylenamidopropyl betaine, alkyl dimethyl amine oxide.

The pH value of the emulsifier system can be adjusted by addition of acid or base, such as hydrochloric acid, sulfuric acid, sodium hydroxide or potassium hydroxide. Usually a pH value in the range of from pH 5 to pH 9 is desired.

The emulsifier system is slightly agitated at a temperature in a range of from 0°C and 60°C, preferably in a range of from 20°C to 50°C before the oil phase is added to the emulsifier system, so that the surfactants form micelles in the aqueous phase. In a particularly preferred embodiment of the described method the emulsifier system is slightly heated to a temperature in the range of from 30°C and 45°C. The emulsifier system is preferably stirred with low shear. The stirring rate for low shear stirring is usually below 10000 rpm, particularly below 4000 rpm.

In the next step an oil phase comprising at least one superhydrophobic monomer and/or a hydrophobic monomer is added to the slightly stirred emulsifier system. The addition of the oil phase is carried out at temperatures between 0°C and 50°C, preferably between 15°C and 30°C. The oil phase is added slowly so that the viscosity of the reaction mixture increases continuously and the mixtures becomes gel-like.

Superhydrophobic monomers are monomers with a solubility in water at 20°C of less than 0,0001 wt.-%, particularly less than 0,00003 wt.-%, which is three orders of magnitude lower than the solubility of styrene in water.

Examples for superhydrophobic monomers are (C₁₀-C₂₆)-alkyl acrylates, or (C₁₀-C₂₆)-alkyl methacrylates such as lauryl acrylate, lauryl methacrylate, myristyl acrylate, myristyl methacrylate, palmityl acrylate, palmityl methacrylate, stearyl acrylate, stearyl methacrylate, and isobornyl acrylate. Further superhydrophobic monomers are fluorinated acrylate monomers.

Hydrophobic monomers according to the present invention are non-superhydrophobic monomers with a solubility in water of up to 5 wt.-% at 20°C. Monomers with a water-solubility at 20°C of at least 5 wt.-% are hydrophilic monomers according to the present invention. Examples for hydrophobic monomers are styrene, methyl acrylate, ethyl acrylate, n-propyl acrylate, iso-propyl acrylate, n-butyl acrylate, sec.-butyl acrylate, iso-butyl acrylate, tert.-butyl acrylate, n-hexyl acrylate, 2-ethyl hexyl acrylate, methyl methacrylate, ethyl methylacrylate, n-propyl methacrylate, iso-propyl methacrylate, n-butyl methacrylate, sec.-butyl methacrylate, iso-butyl methacrylate, tert.-butyl methacrylate, n-hexyl methacrylate, 2-ethyl hexyl methacrylate, benzyl acrylate, phenyl acrylate, benzyl methacrylate, phenyl methacrylate, vinyl acetate, and vinyl esters of versatic acid, such as VeoVa 9, 10 and 11, and ethylenic unsaturated silane monomers, such as metacryloxysilanes and vinylsilanes.

The superhydrophobic monomers and hydrophobic monomers are usually oils. They can be used as oil phase as such in the described method. The oil phase may comprise an organic solvent in addition. Suitable solvents are for example xylene, toluene, or solvent naphta. But preferably no solvent is present in the oil phase.

With the method described herein also miniemulsions comprising further co-monomers are available. The co-monomers are part of the oil-phase and are preferably added as monomer mixture together with the superhydrophobic and/or hydrophobic monomers to the emulsifier system. But the monomers can be also added separately.

Co-monomers are monomers different from superhydrophobic monomers and include hydrophilic monomers as well as hydrophobic monomers, including the hydrophobic monomers mentioned above. Typical co-monomers are hydrophobic and hydrophilic ethylenically unsaturated monomers.

The described method is particularly useful for the preparation of miniemulsions comprising superhydrophobic monomers and/or hydrophobic monomers suitable for the preparation of polymer dispersions by free-radical polymerization.

The oil phase optionally contains a stabilizer. Typical stabilizers are alkanes with 10 to 22 carbon atoms, preferably tetradecane, pentadecane, hexadecane, heptadecane, and octadecane. Alternatively, long alkyl chain alcohols can be employed as stabilizer in the oil phase. Typically long alkyl chain alcohols are (C₁₀-C₃₀-alkanols, such as for example cetyl alcohol. Polymeric stabilizers, such as polystyrene (PS), poly methyl methacrylate (PMMA) or Polyvinyl acetate (PVAc) can be used, also. The role of the stabilizer is to avoid diffusion driven changes of the droplet size distribution of the monomer emulsion.

The oil phase comprises from 5 wt.-% to 100 wt.- superhydrophobic monomers or hydrophobic monomers. Preferably the amount of superhydrophobic monomers and hydrophobic monomers in the oil phase is at least 20 wt.-%, more preferably more than 50 wt.-%, particularly more than 75 wt.-%. The content of optional co-monomers is the range of 0 wt.-% to 95 wt.-%, typically in the range of 1 wt.-% to 80 wt.-%, preferably between 5 wt.-% and 50 wt.-%. The content of the optional stabilizer in the oil phase is up to 10 wt.-%, preferably between 0.1 wt.-% and 5 wt.-%.

To the oil phase certain amounts of the emulsifiers described above can be added additionally. The amount of the additional emulsifier, based on the total amount of the oil phase should be less than 10 wt.-%, particularly less than 5 wt.-%.

The gel-like mixture comprising the emulsifier system and the oil phase is subsequently diluted with an aqueous phase at a temperature between 0° and 50°C, preferably between 15°C and 30°C to achieve the desired miniemulsion. The dilution is carried out under slightly agitation (low shear stirring). The aqueous phase comprises preferably at least 50 wt.-% of water. A water content of the aqueous phase in the range of from 70 wt.-% to 100 wt.-% is preferred. Preferably deionized water (DI water) is used.

Typically the amount of emulsifier system, based on the total amount of the obtained miniemulsion, provided in step a) of the described method is in the range of from 1 wt.-% to 25 wt.-%, a range of from 3 wt.-% to 15 wt.-% is preferred. The amount of the oil phase added to the emulsifier system according to step b) of the method is typically in the range of from 5 wt.-% to 70 wt.-%, preferably in the range of from 15 wt.-% to 50 wt.-%. The aqueous phase in dilution step c) is used in an amount of from 25 wt.-% to 94 wt.-%, preferably in the range of from 40 wt.-% to 80 wt.-%, based on the total amount of the miniemulsion.

Phase transfer agents or polymer seeds are not required for the preparation of miniemulsions with the inventive method. Thus, typically no phase transfer agent or polymer seed added to the emulsifier system, the oil phase or during the dilution step of the inventive method. Such compounds are also usually not applied in the subsequent polymerization of the miniemulsion. However, small amounts of polymer seed, usually less than 5 wt.-% based on the total amount of monomers, can be added to the miniemulsion in method step a), b) or c) or during the polymerization of the miniemulsion, if desired.

During the preparation or prior to the polymerization of the described miniemulsion a buffer system can be added. Known buffers are for example carbonate, phosphate, and/or borate buffers, such as potassium or sodium hydrogen phosphate or dihydrogenphosphate buffers. The buffer should be particularly compatible with the initiator system used in the free-radical polymerization of the miniemulsion.

Further chain transfer agents can be added during or after the preparation of the miniemulsion. Typical chain transfer agents are selected from thiols, disulfides, etc. Further examples are benzene, toluene, isopropyl benzene, isopropanol, chloroform, carbon tetrachloride or 1 butane thiol.

Without wishing to be bound by theory, the initial addition of the oil phase to the aqueous emulsifier leads to the formation of a swollen lamellar phase, which evolves under further oil-phase addition into a highly viscous and typically isotropic phase (gel formation, gel-like phase), most probably consisting of dense packed small oil droplets surrounded by a continuous aqueous phase, which gets into a miniemulsion upon dilution with water. The formation of a gel during the addition of the oil phase to the emulsifier system and the formation of a miniemulsion by diluting the gel-like mixture can be verified for example by X-ray scattering.

A further embodiment of the present invention are miniemulsions obtainable by the described method.

The obtained miniemulsions are oil-in-water miniemulsion comprising from
5 wt.-% to 70 wt.-%, preferably from 10 wt.-% to 60 wt.-% of an oil phase which comprises from
   5 wt.-% to 100 wt.-% of one or more superhydrophobic monomers or hydrophobic monomers and 0 wt.-% to 95 wt.-% of non-superhydrophobic monomers, stabilized with
1 wt.-% to 25 wt.-%, preferably from 3 wt.-% to 15 wt.-% of an emulsifier system comprising from
   10 wt.-% to 90 wt.-% of at least one non-ionic surfactant selected from the group of saturated branched alcohols having 7 to 30 carbon atoms which are alkoxylated with 4 to 20 alkyloxy groups selected from the group consisting of ethylene oxide, propylene oxide and butylene oxide or mixtures thereof, or of a mixture of surfactants consisting of at least 20 wt.-% and less than 100 wt.-% of the at least one non-ionic surfactant and more than 0 wt.-% to 80 wt.-% of at least one anionic surfactant, and from 10 wt.-% to 90 wt.-% water, and
25 wt.-% to 94 wt.-%, preferably from 40 wt.-% to 80 wt.-% of additional water, wherein
the monomer droplets have a mean droplet diameter D50 value in the range of from 50 to 1000 nm.

Miniemulsions according to the present invention are emulsions in which the particles of the dispersed phase have diameters in the range of from 50 nm to 1000 nm. The oil-in-water emulsions of the present invention comprise preferably droplets with a mean diameter in the range of from 50 nm to 800nm, preferably from 50 to 500 nm and particularly preferred from 100 to 300 nm.

The mean diameter is determined by laser light scattering, for example by using a Horiba LA 940 or Mastersizer 3000 from Malvern using the "Mie Scattering Theory" evaluation.

In case of droplets having axes of different length, such as droplets having the shape of an ellipsoid or of a disk, the largest axis determines the mean diameter.

The droplets of the present invention have a narrow particle size distribution of Gaussian shape. Preferably the standard deviation of the droplet size distribution is between 10 % and 120 % of the mean diameter. Preferably the standard deviation is between 20 % and 90 %

The miniemulsions according to the present invention are stable for at least 24 hours, often for several days, more often for several weeks. The stability of the miniemulsions can be assessed, for example, by centrifugation analysis of the samples. Analytical devices such as LUMiFuge or LUMiSizer are suitable to perform advanced assessment of miniemulsion stability by tracking the light transmission profile along the sample while it is centrifuged, so any phase separation and/or agglomeration process can be detected.

The miniemulsions of the present invention are used for the preparation of polymer dispersions, wherein the dispersed polymers comprising monomeric units derived from superhydrophobic and/or hydrophobic monomers.

The monomer or mixture of monomers in the dispersed oil phase of the miniemulsions can be polymerized under free radical polymerization conditions, preferably in the presence of a free radical initiator. The polymerization initiator may be either a water-soluble or an oil soluble compound.

Suitable free radical initiators are well known in the art and comprise for example, organic peroxides such as tert-butyl hydroperoxide, benzoyl peroxide, lauroyl peroxide, 2, 5-dimethyl 2,5-di (2-ethylhexanoylperoxy) hexane and dicumyl peroxide; inorganic persulfates such as potassium, sodium and/or ammonium persulfate ; and azo initiators such as azobis- (isobutyro nitrile) (AIBN) and azobis (1-cyclohexanecarbonitrile) ; and/or redox pairs such as those comprising Fe²⁺/H₂0₂, and/or K₂S₂0₈/Fe²⁺ either paired with for example ascorbic acid and/or one or more bisulfites.

The free-radical initiator may be added after, before and/or during the miniemulsification process described above. Preferably, the free-radical initiator is added after miniemulsification is completed.

The polymerization may be carried out over a broad temperature range depending on the choice of initiator, preferably from about 20 to about 90 C, more preferably from about 25 to about 80 C, for example about 70 C.

The polymerization is usually conducted over a period from about 10 min to about 24 hrs, more usually from about 2 to about 10 hours, most usually from about 2 to about 6 hours.

The aqueous polymer dispersions produced from the monomer miniemulsions of this invention may form after evaporation of the aqueous solvent polymer films, therefore they shall find use as binders in paints and coatings. Due to the incorporation of superhydrophobic monomers, the binders may be used to enhance the barrier properties of paints and coatings, especially their water resistance.

### Examples:

Hostaphat 1306 = Phosphate ester of iso-tridecanol ethoxylate
Genapol X080 = Iso-tridecanol ethoxylate (8 EO)
Genapol X060 = Iso-tridecanol ethoxylate (6 EO)
Genapol X020 = Iso-tridecanol ethoxylate (2 EO)
Genapol LA080 = Lauryl alcohol ethoxylate (8 EO)
Genapol 0080 = Oleyl alcohol ethoxylate (8 EO)
Brij O10 = Oleyl alcohol ethoxylate (10 EO)
Brij S20 = Stearyl alcohol ethoxylate (20 EO)
Luperox® TBH7OX = tert-Butyl hydroperoxide solution

### Example 1: Preparation of a miniemulsion of lauryl methacrylate

6.2 g of Phase A (emulsifier system 1 (ES1)) are poured in a 250 ml glass beaker and slightly warmed on a heating plate to a temperature of 35 to 40°C. The beaker is then taken from the heating plate and put on the bench at room temperature. The phase A is stirred with a frame stirrer powered with an IKA Eurostar 20 Digital overhead motor at 450 rpm.

**Table 1: Phase A**

| Emulsifier System (ES1) | wt.-% |
|---|---|
| Glycerol | 26.1 |
| Water | 32.7 |
| Iso-tridecanol ethoxylate (8 EO) (Genapol X080) | 30.2 |
| Phosphate ester of iso-tridecanol ethoxylate (Hostaphat 1306) | 11.0 |

pH value is adjusted to 7 with NaOH (50%)

Phase B containing 20.0 g lauryl methacrylate and 0.8 g hexadecane are added dropwise (addition ratio 800 ml/hour) at room temperature to the stirred Phase A so carefully that viscosity increases continuously and the mixture gets gel-like; at this point the rotation speed of the stirrer is increased to 900 rpm and the dropwise addition of Phase B is continued until is completed, yielding an almost transparent highly viscous gel, which is stirred for 30 seconds at a rotation speed of 1800 rpm.

73.0 g of desionized water are added (addition rate 7300 ml/hour) at room temperature to the gel under stirring to get a 20 wt% monomer miniemulsion with a D₅₀ value for the droplet size distribution (DSD) of 180 nm with a standard deviation of 60 nm.

Method for the determination of the mean droplet size (mean diameter) D50 value and instability index:
The particle size analysis of the samples (miniemulsions and polymer dispersions) is carried out in a particle analyzer Horiba LA-960, using a refractive index of 1.48 - 0.00i for the disperse phase and 1.33 for the aqueous continuous phase. The particle size distribution (volume) is calculated from the analysis of the scattering data making use of the Mie scattering theory according to ISO 13320. The values of the mean droplet size (mean diameter) D50 and the standard deviation are automatically calculated from the above mentioned distribution by the software of the instrument.

The stability of the samples (miniemulsions and polymer dispersions) was assessed in terms of the instability index calculated from the changes in light transmission at 870 nm along the sample during centrifugation performed in a LUMiSizer instrument. The samples were filled in polyamide cuvettes and centrifuged at 4000 rpm for 2.1 hours. The instability index was calculated automatically by the software of the instrument. The instability index can take values from 0 to 1, the lower the instability index the better the stability of the sample.

Polymerization of the miniemulsion obtained from example 1:
The miniemulsion obtained as described in example 1 was heated under a flow of nitrogen to 70°C. 1.58 g of aqueous Luperox® TBH7OX (17.7 wt.-% in water) and 1.65 g of aqueous ascorbic acid (12.0 wt.-% in water) were added to the miniemulsion and the mixture was stirred for 2 hours at 70°C. The initiation of the polymerization reaction was detected after addition of the initiator system by rapid increase of the temperature of the reaction mixture. After polymerization, a low viscous bluish polymer dispersion was obtained.

### Examples 2-10: Variation of emulsifier system:

The emulsions of examples 2-9 are prepared as in example 1, wherein the total amount of surfactant in phase A is 41.2 wt.-%. The mass ratio of the surfactant mixture used in the examples is provided in Table 2. (CoV in Table 2 means Coefficient of Variation = ratio of the standard deviation to the mean D50 value.)

**Table 2:**

| Example | Non-ionic surfactant: anionic surfactant (mass ratio) | miniemulsion formation (D50 - CoV) |
|---|---|---|
| 2 (comparative) | Genapol X020 (75) : Hostaphat 1306 (25) | no (phase separation) |
| 3 | Genapol X060 (75) : Hostaphat 1306 (25) | yes (292 nm - 41%) |
| 4 (comparative) | Genapol LA080 (75) : Hostaphat 1306 (25) | no (10 µm) |
| 5 (comparative) | Genapol O080 (75) : Hostaphat 1306 (25) | no (10 µm) |
| 6 (comparative) | Genapol O100 (75) : Hostaphat 1306 (25) | no (phase separation) |
| 7 | Genapol X080 (25) : Hostaphat 1306 (75) | yes (192 nm - 36%) |
| 8 | Genapol X080 (100) : Hostaphat 1306 (0) | yes (228 nm - 39%) |
| 9 (comparative) | Genapol X080 (0) : Hostaphat 1306 (100) | no (phase separation) |

### Examples 10 - 34

The emulsions of example 10 to 34 are prepared as in example 1, wherein different emulsifier systems (ES1 - ES4) and monomer compositions are tested. In comparative example 13 only DI water is provided as phase A. In comparative example 14 no phase A is provided. The emulsifier is added to Phase B which is diluted with DI water as described in examples 1. The emulsifier systems ES2, ES3 and ES4 are listed in Table 3, 4 and 5. The oil phase comprising different monomer compositions (Phase B) are mentioned in Table 6

**Table 3:**

| Emulsifier System (ES2) | wt.-% |
|---|---|
| Glycerol | 26.1 |
| Water | 31.5 |
| Iso-tridecanol ethoxylate (8 EO) (Genapol X080) | 30.2 |
| Emulsogen EPA 079 (C11-Oxo alcohol-heptylglycol ether sulphate sodium salt | 12.2 |

pH value is adjusted to 7 with NaOH (50%)

**Table 4:**

| Emulsifier System (ES3) | wt.-% |
|---|---|
| Urea | 19.8 |
| Water | 24.7 |
| Ethanol | 24.4 |
| Iso-tridecanol ethoxylate (8 EO) (Genapol X080) | 22.8 |
| Phosphate ester of iso-tridecanol ethoxylate (Hostaphat 1306) | 8.3 |

pH value is adjusted to 7 with NaOH (50%)

**Table 5:**

| Emulsifier System (ES4) | wt.-% |
|---|---|
| Water | 53.2 |
| Iso-tridecanol ethoxylate (8 EO) (Genapol X080) | 34.3 |
| Phosphate ester of iso-tridecanol ethoxylate (Hostaphat 1306) | 12.5 |

pH value is adjusted to 7 with NaOH (50%)

**Table 6:**

| | | | | | Example 10 (comparative) | Example 11 (comparative) | Example 12 (comparative) | Example 13 (comparative) | Example 14 (comparative) | Example 15 (comparative) |
|---|---|---|---|---|---|---|---|---|---|---|
| Phase A, 40°C | | | | | wt.-% | wt.-% | wt.-% | wt.-% | wt.-% | wt.-% |
| Brij 010 | | | | | 2.50 | 5.50 | | | | 2.50 |
| Brij S20 | | | | | | | 2.20 | | | |
| DI water | | | | | 2.50 | 5.50 | 2.20 | 2.20 | | 1.25 |
| Glycerol | | | | | | | | | | 1.25 |

| Phase B, 20°C | | | | | | | | | | |
|---|---|---|---|---|---|---|---|---|---|---|
| Brij S20 | | | | | | | | 2.20 | 2.20 | |
| Lauryl Methacrylate | | | | | 17.00 | 38.00 | 20.00 | 20.00 | 20.00 | 17.00 |
| Hexadecane | | | | | | | 0.80 | 0.80 | 0.80 | |

| Phase C, 20°C | | | | | | | | | | |
|---|---|---|---|---|---|---|---|---|---|---|
| DI Water | | | | | 78.00 | 51.00 | 74.80 | 74.80 | 77.00 | 78.00 |

| | Example 1 | Example 16 | Example 17 | Example 18 | Example 19 | Example 20 | Example 21 | Example 22 | Example 23 | Example 24 |
|---|---|---|---|---|---|---|---|---|---|---|
| Phase A, 40°C | | | | | | | | | | |
| ES1 | 6.20 | 6.20 | 6.20 | 12.40 | 15.50 | 6.20 | 6.20 | 12.40 | | |
| ES2 | | | | | | | | | 6.20 | 12.40 |
| ES3 | | | | | | | | | | |
| ES4 | | | | | | | | | | |

| Phase B, 20°C | | | | | | | | | | |
|---|---|---|---|---|---|---|---|---|---|---|
| Lauryl Methacrylate | 20.00 | 30.00 | 50.00 | 40.00 | 50.00 | 20.00 | 40.00 | 40.00 | 20.00 | 40.00 |
| Stearyl Methacrylate | | | | | | | | | | |
| Styrol | | | | | | | | | | |
| Butylacrylate | | | | | | | | | | |
| Hexadecane | 0.80 | 1.20 | 2.00 | 1.60 | 2.00 | | | | 0.80 | 1.60 |

| Phase C, 20°C | | | | | | | | | | |
|---|---|---|---|---|---|---|---|---|---|---|
| DI Water | 73.00 | 62.60 | 41.80 | 46.00 | 32.50 | 73.80 | 53.80 | 47.60 | 73.00 | 46.00 |

| | Example 25 | Example 26 | Example 27 | Example 28 | Example 29 | Example 30 | Example 31 | Example 32 | Example 33 | Example 34 |
|---|---|---|---|---|---|---|---|---|---|---|
| Phase A, 40°C | | | | | | | | | | |
| ES1 | 6.20 | 6.20 | 12.40 | 12.40 | 6.20 | 6.20 | 12.40 | | | |
| ES2 | | | | | | | | | | |
| ES3 | | | | | | | | 6.20 | | |
| ES4 | | | | | | | | | 5.20 | 5.20 |

| Phase B, 20°C | | | | | | | | | | |
|---|---|---|---|---|---|---|---|---|---|---|
| Lauryl Methacrylate | | | | | 6.67 | 13.33 | 13.33 | 48.10 | 20.00 | 20.00 |
| Stearyl Methacrylate | 20.00 | 40.00 | 40.00 | | | | | | | |
| Styrol | | | | 20.00 | 6.67 | 13.33 | 13.33 | | | |
| Butylacrylate | | | | 20.00 | 6.67 | 13.33 | 13.33 | | | |
| Hexadecane | 0.80 | 1.60 | 1.60 | 1.60 | 0.80 | 1.60 | 1.60 | 1.90 | 0.80 | 0.80 |

| Phase C, 20°C | | | | | | | | | | |
|---|---|---|---|---|---|---|---|---|---|---|
| DI Water | 73.00 | 52.20 | 46.00 | 46.00 | 73.00 | 52.20 | 46.00 | 41.80 | 73.00 | 73.00 |

**Table 7**

| Exa. | droplet size distribution D₅₀ (standard deviation) | Instability Index | droplet size distribution D₅₀ (polymer) (standard deviation) | Instability Index (polymer) |
|---|---|---|---|---|
| 1 | 0.18 µm (0.06 µm) | 0.568 | 0.107 µm (0.03 µm) | 0.002 |
| 10 | 29.25 µm (14.50 µm) | 0.928 | | |
| 11 | 37.04 µm (15.66 µm) | 0.766 | | |
| 12 | 44.85 µm (23.44 µm) | | | |
| 13 | 34.33 µm (24.83 µm) | | | |
| 14 | 5.39 µm (3.70 µm) | | | |
| 15 | 24.08 µm (14.10 µm) | 0.884 | | |
| 16 | 0.22 µm (0.08 µm) | 0.373 | 0.122 µm (0.04 µm) | 0.003 |
| 17 | 0.26 µm (0.29 µm) | 0.005 | | |
| 18 | 0.18 µm (0.06 µm) | 0.002 | 0.099 µm (0.03 µm) | 0.207 |
| 19 | 0.18 µm (0.06 µm) | <0.001 | 0.101 µm (0.03 µm) | 0.151 |
| 20 | 0.18 µm (0.06 µm) | 0.613 | 0.114 µm (0.04 µm) | 0.005 |
| 21 | 0.28 µm (0.15 µm) | 0.371 | | |
| 22 | 0.18 µm (0.06 µm) | 0.142 | 0.110 µm (0.04 µm) | 0.095 |
| 23 | 0.24 µm (0.09 µm) | 0.608 | 0.109 µm (0.49 µm) | 0.210 |
| 24 | 0.22 µm (0.08 µm) | 0.856 | 0.108 µm (0.04 µm) | 0.539 |
| 25 | 0.16 µm (0.05 µm) | 0.425 | 0.147 µm (0.05 µm) | 0.028 |
| 26 | 0.23 µm (0.25 µm) | 0.014 | | |
| 27 | 0.16 µm (0.05 µm) | 0.001 | 0.158 µm (0.06 µm) | 0.001 |
| 28 | 0.17 µm (1.18 µm) | 0.143 | 0.101 µm (0.03 µm) | 0.014 |
| 29 | 0.21 µm (0.08 µm) | 0.765 | 0.102 µm (0.03 µm) | 0.009 |
| 30 | 0.25 µm (0.14 µm) | 0.275 | 0.139 µm (0.05 µm) | 0.001 |
| 31 | 0.20 µm (0.07 µm) | 0.132 | 0.098 µm (0.07 µm) | 0.035 |
| 32 | 0.21 µm (0.08 µm) | 0.428 | 0.191 µm (0.07 µm) | |
| 33 | 0.16 µm (0.06 µm) | 0.297 | 0.100 µm (0.03 µm) | |
| 34 | 0.17 µm (0.06 µm) | 0.173 | 0.100 µm (0.03 µm) | |

From the results presented in Table 2, Example 3, 7 and 8 and Table 7, Example 1 and 16-34 becomes clear that miniemulsions of superhydrophobic monomers or hydrophobic monomers are easily available. The mean diameter (D50) of the miniemulsions of these examples is in the range of from 160 nm to 280 nm. High shear stirring, high pressure homogenization or phase inversion methods as well as toxic solvents, polymeric seeds or phase transfer agents are not required.

In comparative examples 3, 4, and 5 (Table 2) and comparative examples 10-15 (Table 6 and 7) linear alkanol ethoxylates are employed as emulsifier. The emulsifier system of comparative example 2 comprises a branched iso-tridecanol ethoxylate having 2 EO units, only. No miniemulsions are obtained with these emulsifiers in contrary to example 1, 3, 7, 8, and 16 - 34 wherein a branched iso-tridecanol ethoxylate with 6 or 8 EO units is used as emulsifiers.

In comparative example 14 the PIC procedure is used and water (phase C) is added to the oil (monomer) phase where the emulsifier is dissolved. No miniemulsion is formed

Anionic surfactants, such as Hostaphat 1306, are optional emulsifiers (example 8) but can be present in high amounts (example 7). By an emulsifier system comprising Hostaphat 1306, only, no miniemulsion is obtained (comparative example 9).

## Claims

1. A method for preparing an oil-in-water miniemulsion comprising monomer droplets with a mean diameter in the range of from 50 to 1000 nm, wherein the method comprises the steps of
(a) stirring an emulsifier system comprising
(i) at least one non-ionic surfactant selected from the group of saturated branched alcohols having 7 to 30 carbon atoms which are alkoxylated with 4 to 20 alkyloxy groups selected from the group consisting of ethylene oxide, propylene oxide and butylene oxide or mixtures thereof, or a mixture of surfactants consisting of at least 20 wt.-% and less than 100 wt.-% of the at least one non-ionic surfactant and more than 0 wt.-% to 80 wt.-% of at least one anionic surfactant, and
(ii) water,
and adjusting the temperature of the emulsifier system to a range between 0°C and 50°C,
(b) adding an oil phase to the emulsifier system (a), wherein the oil phase comprises at least 5 wt.-% to 100 wt.-% of one or more superhydrophobic monomers or hydrophobic monomers and 0 wt.-% to 95 wt.-% of non-superhydrophobic monomers, until gel formation occurs, and
(c) diluting the gel obtained in step (b) with an aqueous phase under stirring at a temperature between 0°C and 50°C, wherein a miniemulsion comprising the superhydrophobic monomers is obtained.

2. A method according to claim 1, wherein the at least one non-ionic surfactant is a branched (C₉-C₂₁)-alkanol having a terminal hydroxyl group alkoxylated with 4 to 20 alkoxy groups selected from the group consisting of ethylene oxide, propylene oxide and butylene oxide, wherein the branched alkyl residue of the alkanol comprises one, two or three alkyl side groups selected from the group of methyl and ethyl.

3. A method according to claim 1, wherein the non-ionic surfactant is selected from a compound of formula (I)
H-(OA)ₘ-O-(CR'₂)ₙ-CR₃ (I)
wherein
at least one of the residues R' and/or at least two of the residues R are independently methyl, ethyl, propyl or butyl and the remaining residues R and R' are hydrogen, and wherein the number of carbon atoms of the residue
-(CR'₂)-CR₃ is from 7 to 30,
the groups (OA) are selected from the group of ethylene oxide, propylene oxide, butylene oxide and mixtures thereof,
m is an integer from 4 to 20, and
n is an integer from 7 to 16.

4. A method according to any one of claims 1 to 3, wherein the non-ionic surfactant is selected from a compound of formula (II)
H-(O-C₂H₄)ₘ-O-(CH₂)ₙ-CHR₂ (II)
wherein
both residues R are independently methyl or ethyl,
m is an integer from 6 to 12, and
n is an integer from 9 to 13.

5. A method according to any one of claims 1 to 4, wherein the at least one anionic surfactant is selected from the group of phosphate esters of alkoxylated aliphatic or aromatic alcohols.

6. A method according to any one of claims 1 to 5, wherein the at least one anionic surfactant is selected from the group of phosphate esters of a compound of formula (III)
H-(OA)ₘ-O-(CR₂)ₙ-CR₃ (III)
wherein
one, two or three of the residues R are independently methyl or ethyl and the remaining residues R are hydrogen or all residues R are hydrogen,
the groups (OA) are selected from the group of ethylene oxide, propylene oxide, butylene oxide and mixtures thereof,
m is an integer from 2 to 20, and
n is an integer from 5 to 21.

7. A method according to any one of claims 1 to 6, wherein the oil phase comprises at least one superhydrophobic monomer selected from the group consisting of (C₁₀-C₂₆)-alkyl acrylate and (C₁₀-C₂₆)-alkyl methacrylate and fluorinated acrylate monomers.

8. A method according to any one of claims 1 to 7, wherein the superhydrophobic monomers are selected from the group consisting of lauryl acrylate, lauryl methacrylate, myristyl acrylate, myristyl methacrylate, palmityl acrylate, palmityl methacrylate, stearyl acrylate, stearyl methacrylate, isobornyl acrylate, and fluorinated acrylate monomers.

9. A method according to any one of claims 1 to 8, wherein the oil phase comprises up to 95 wt.-% of non-superhydrophobic co-monomers.

10. A method according to any one of claims 1 to 9, wherein the pH value of emulsifier system is adjusted to 5 to 9.

11. A method according to any one of claims 1 to 10, wherein the emulsifier systems comprises at least one co-solvent selected from the group of methanol, ethanol, propanol, butanol, benzyl alcohol, ethylene glycol, di-ethylene glycol, propylene glycol, di-propylene glycol, glycerol, polyethylene glycol, polypropylene glycol, and mono-methyl ether or di-methyl ether of ethylene glycol or propylene glycol.

12. A method according to any one of claims 1 to 11, wherein the oil phase comprises a stabilizer.

13. A method for preparing a polymer dispersion comprising the method steps of any one of claims 1 to 12, wherein the miniemulsion comprising at least 5 wt.-% of at least one superhydrophobic monomer is polymerized.

14. A miniemulsion obtainable by a method according to anyone of claims 1 to 12 or a polymer dispersion obtainable by a method according to claim 13.
